# EUROPÄISCHE PATENTSCHRIFT

(11) **EP 3 609 551 B1**
(45) Veröffentlichungstag und Bekanntmachung des Hinweises auf die Patenterteilung: **07.06.2023**
(21) Anmeldenummer: 18717040.2
(22) Anmeldetag: 10.04.2018
(51) Int. Cl.: A61M 1/16, H01L 23/473

(54) **VORRICHTUNG ZUR EXTRAKORPORALEN BLUTBEHANDLUNG UND VERFAHREN ZUM BETREIBEN EINER EXTRAKORPORALEN BLUTBEHANDLUNGSVORRICHTUNG**
DEVICE FOR EXTRACORPOREAL BLOOD TREATMENT AND METHOD FOR OPERATING AN EXTRACORPOREAL BLOOD TREATMENT DEVICE
DISPOSITIF DE TRAITEMENT EXTRACORPOREL DU SANG ET PROCÉDÉ DE FONCTIONNEMENT D'UN DISPOSITIF DE TRAITEMENT EXTRACORPOREL DU SANG

(30) Priorität: 11.04.2017 DE 102017003508
(43) Veröffentlichungstag der Anmeldung: 19.02.2020
(73) Patentinhaber: Fresenius Medical Care Deutschland GmbH, 61352 Bad Homburg v.d.H (DE)
(72) Erfinder: GLASER, Benedict, 97421 Schweinfurt (DE)
(74) Vertreter: Oppermann, Frank
(86) Internationale Anmeldenummer: PCT/EP2018/059207
(87) Internationale Veröffentlichungsnummer: WO 2018/189207

(56) Entgegenhaltungen:
- DE-A1- 10 056 172
- US-A1- 2011 309 019
- US-A1- 2016 030 660

## Beschreibung

Die Erfindung betrifft eine Vorrichtung zur extrakorporalen Blutbehandlung mit einem mehrere Leitungen umfassenden Hydrauliksystem zur Bereitstellung von Dialysat für einen Dialysator bzw. eine Dialysebehandlung, wobei bei der Behandlung der Dialysator von Dialysat durchströmt wird und/oder das Dialysat als Substituat einem extrakorporalen Blutschlauchsystem zugeführt wird, und mit einem mindestens ein, normalerweise mehrere, elektrische Bauteile umfassenden Steuerungssystem. Darüber hinaus betrifft die Erfindung ein Verfahren zum Betreiben einer Vorrichtung zur extrakorporalen Blutbehandlung in einem Betriebsmodus zur Durchführung einer Heiß-Desinfektion, wobei die Vorrichtung zur extrakorporalen Blutbehandlung ein mehrere Leitungen umfassendes Hydrauliksystem zur Bereitstellung von Dialysat für einen Dialysator bzw. eine Dialysebehandlung aufweist, wobei bei der Behandlung der Dialysator von Dialysat durchströmt wird oder das Dialysat als Substituat einem extrakorporalen Blutschlauchsystem zugeführt wird, und die ein mindestens ein, normalerweise mehrere, elektrische Bauteile umfassendes Steuerungssystem aufweist, wobei das Dialysat unter Verwendung einer der Vorrichtung zur extrakorporalen Blutbehandlung zugeführten Flüssigkeit hergestellt wird.

Bei der Dialyse durchströmt das zu behandelnde Blut in einem extrakorporalen Blutkreislauf die Blutkammer eines durch eine semipermeable Membran in die Blutkammer und eine Dialysatkammer unterteilten Dialysators, während Dialysat die Dialysatkammer des Dialysators durchströmt. Der extrakorporale Blutkreislauf umfasst eine Blutzuführleitung, die zu der Blutkammer führt, und eine Blutabführleitung, die von der Blutkammer abgeht. Das Hydrauliksystem der Vorrichtung zur extrakorporalen Blutbehandlung weist eine zu dem Dialysator führende Leitung zum Zuführen von frischem Dialysat und eine von dem Dialysator abgehende Leitung zum Abführen von gebrauchtem Dialysat zu einem Ablauf auf. Zum Fördern der Flüssigkeiten sind Pumpen vorgesehen. Die Steuerung sämtlicher Komponenten der Vorrichtung zur extrakorporalen Blutbehandlung erfolgt mit einem Steuerungssystem, das mehrere elektrische Bauteile umfasst. Das Steuerungssystem kann aus einer oder mehreren Baugruppen bestehen. Diese Beschreibung ist exemplarisch für eine Dialyse. Im Sinne der vorliegenden Erfindung umfasst der Begriff Dialyse auch den Fall einer Hämofiltration, bei der das Dialysat nicht der Dialysatkammer zugeführt wird, sondern als Substituat der Blutzuführungsleitung und/oder der Blutabführungsleitung zugeführt wird und über den Dialysator Flüssigkeit entzogen wird. Im Sinne der vorliegenden Erfindung umfasst der Begriff Dialyse alle Kombination der genannten Verfahren.

Das Dialysat kann in der Dialysemaschine aus Permeat (Reinwasser) und einem oder mehreren Konzentraten hergestellt werden. Für die Zuführung des Permeats weisen die Dialysemaschinen einen Wasseranschluss auf. Die Aufbereitung des Dialysats erfolgt in dem Hydrauliksystem der Maschine. Es sind aber auch sogenannte "Bed-Side-Stations" bekannt und stellen Ausführungsformen das, bei denen das Dialysat der Dialysemaschine von außen zugeführt wird.

Wenn im Folgenden von "Wasser" oder "Reinwasser" die Rede ist, wird darunter auch Dialysat oder eine Spülflüssigkeit verstanden, das bzw. die der Dialysemaschine von außen zugeführt wird oder in der Dialysemaschine bereitgestellt wird. "Wasser" und "Reinwasser" können im Sinne der vorliegenden Erfindung die gleiche Bedeutung oder eine unterschiedliche Bedeutung haben. Ob das Wasser auch ein Reinwasser sein kann, ergibt sich für den Fachmann aus dem technischen Zusammenhang. Im Zweifel sind die Begriffe als Synonyme zu verstehen.

Die Dialysemaschinen werden zunehmend kompakt aufgebaut. Die Gründe für diese Entwicklung liegen in der verbesserten Transportmöglichkeit und der Raumeinsparung bei beengten Platzverhältnissen in der Klinik oder im Heimdialysebereich. Eine andere Entwicklung ist, dass bei Dialysemaschinen sich vielfach Heißreinigungs- oder Heißdesinfektions-Verfahren durchgesetzt haben und diese Verfahren noch weiter an Bedeutung gewinnen. Durch die genannten Verfahren lässt sich u.a. der Einsatz von Chemikalien reduzieren. Bei der Heiß-Desinfektion zirkuliert im Hydrauliksystem auf eine Temperatur von größer als 80°C erhitztes Permeat und/oder mit Desinfektionsmitteln versetzes Permeat. Die Begriffe Heiß-Reinigung und Heiß-Desinfektion sind in der vorliegenden Beschreibung synonym zu verstehen soweit sich dem Fachmann aus dem Kontext nicht erschließt, dass es sich um das eine oder andere Verfahren handeln muss.

Grundsätzlich hat eine Desinfektion auch eine reinigende Wirkung, während eine Reinigung nicht zwingend zu einer Desinfektion führen muss. So kann man beispielsweise bei einer deutlich niedrigeren Temperatur reinigen als desinfizieren.

Bei Dialysemaschinen mit kompaktem Aufbau wird das thermische Management eine zunehmende Herausforderung. Insbesondere während der Heiß-Desinfektion kann die Temperatur im Inneren des Gehäuses der Dialysemaschine stark ansteigen. Eine hohe Temperatur im Gehäuseinneren ist für das Erreichen der für die Heiß-Desinfektion notwendigen Temperatur notwendig oder zumindest hilfreich. Die Lufttemperatur im Geräteinneren kann 50°C bis 65°C betragen.

Für die elektrischen Bauteile des Steuerungssystems, beispielsweise Leistungshalbleiter oder Prozessoren, stellt die hohe Lufttemperatur ein Problem dar. Dieses Problem wird noch dadurch verschärft, dass sich in kompakten Dialysegeräten die Elektronik in direkter räumlicher Nähe zu den Komponenten des Hydrauliksystems befindet.

Viele elektrische Bauteile sind nur bis zu einer Temperatur von maximal 45°C/50°C zugelassen. Darüber hinaus reduziert sich die Haltbarkeit elektrischer Bauteile beim Überschreiten bestimmter Temperaturen deutlich. Daher ist eine ausreichende Kühlung der elektrischen Bauteile erforderlich. Die Kühlung elektrischer Bauteile erfolgt im Allgemeinen mit den bekannten Kühlkörpern, auf denen die Bauteile montiert sind. Diese Kühlung hat sich bei den hohen Temperaturen im Gehäuseinneren aber nicht als ausreichend erwiesen.

Zum Stand der Technik gehören daher Dialysemaschinen, die über zusätzliche Lüfter verfügen, die bedarfsgerecht gesteuert werden. Mit den Lüftern kann das thermische Problem gelöst werden. Allerdings ist der Einsatz von Lüftern mit Nachteilen verbunden.

Zunächst verursachen die Lüfter zusätzliche Herstellungskosten. Dabei ist zu berücksichtigen, dass die Lüfter eine nur begrenzte Standzeit haben. Mit den Lüftern wird weiterhin der Staubeintrag in die Geräte erhöht. Darüber hinaus ist ein höherer Geräuschpegel die Folge. Im Übrigen hat sich die Luftkühlung in Ländern mit sehr hohen Außentemperaturen nicht immer als praktikabel erwiesen.

Die US 2011/0309019 A1 beschreibt eine extrakorporale Blutbehandlungsvorrichtung, die über eine Messeinrichtung verfügt, die eine Lichtquelle und zwei Fotodetektoren aufweist. Die US 2011/0309019 A1 befasst sich insbesondere mit dem Problem einer Temperaturdrift der Fotodetektoren während der Blutbehandlung. Die Blutbehandlungsvorrichtung sieht einen Betriebsmodus zur Durchführung einer HeißDesinfektion des Hydrauliksystems vor, bei der eine auf eine vorgegebene Temperatur erwärmte Flüssigkeit durch mindestens einen Teil der Leitungen des Hydrauliksystems strömt. Zur Temperaturstabilisierung wird vorgeschlagen für die Fotodetektoren einen Kühlkörper mit Wasserkühlung vorzusehen, um nach der Durchführung der HeißDesinfektion die Fotodetektoren mit Dialysat zu kühlen. Die US 2011/0309019 A1 betrifft nicht das Problem der Beschädigung elektrischer Bauteile während der Heiß-Desinfektion aufgrund der dort auftretenden hohen Temperaturen.

Der Erfindung liegt daher die Aufgabe zugrunde, eine Vorrichtung zur extrakorporalen Blutbehandlung zu schaffen, bei der die oben genannten Nachteile vermieden werden. Die Aufgabe der Erfindung liegt insbesondere darin, eine Vorrichtung zur extrakorporalen Blutbehandlung bereitzustellen, die auch bei hohen Außentemperaturen zuverlässig arbeitet. Eine weitere Aufgabe der Erfindung liegt darin, ein Verfahren anzugeben, mit dem eine Vorrichtung zur extrakorporalen Blutbehandlung ohne die oben genannten Nachteile betrieben werden kann.

Die Lösung dieser Aufgabe erfolgt erfindungsgemäß mit den Merkmalen der unabhängigen Patentansprüche. Die abhängigen Ansprüche betreffen bevorzugte Ausführungsformen der Erfindung.

Bei der erfindungsgemäßen Vorrichtung zur extrakorporalen Blutbehandlung handelt es sich um eine Blutbehandlungsvorrichtung, die über einen zentralen Anschluss verfügt, um eine Flüssigkeit, insbesondere Permeat (Reinwasser), der Maschine zuführen zu können. Die Flüssigkeit kann für die Herstellung von Dialysat oder die Heiß-Desinfektion verwendet werden. Da die Flüssigkeit zugeführt wird, steht eine ausreichende Flüssigkeitsmenge zur Verfügung. Die Temperatur der Flüssigkeit ist von der Raumtemperatur weitgehend unabhängig und kann mit der zentralen Versorgung auf einen vorgegebenen Wert eingestellt werden, der auch unterhalb einer möglicherweise hohen Raumtemperatur liegen kann.

Die Vorrichtung weist eine Kühleinrichtung zum Kühlen mindestens eines der elektrischen Bauteile des Steuerungssystems auf, die zur Abfuhr der Wärme von den elektrischen Bauteilen die der Vorrichtung zentral zugeführte Flüssigkeit, insbesondere Permeat (Reinwasser), verwendet. Die Kühleinrichtung weist mindestens einen mit einer Flüssigkeit kühlbaren Kühlkörper auf, der mit dem mindestens einem elektrischen Bauteil in einem thermischen Kontakt steht, wobei die Kühleinrichtung mindestens einen Einlass aufweist, der mit dem Anschluss für den Zufluss einer Flüssigkeit in Fluidverbindung steht, und mindestens einen Auslass aufweist, der mit einem Ablauf in Fluidverbindung steht. Der thermische Kontakt ist vorzugsweise ein Berührungskontakt oder ein Orientierungskontakt, bei dem der Kühlkörper unmittelbar beispielsweise eine Leiterplatte berührt oder der Kühlkörper derart ausgerichtet ist, dass seine Kühlwirkung effizient auf die elektrischen Bauteile übertragen wird. Dies kann dadurch erreicht werden, dass der Kühlkörper beispielsweise in einem Abstand von weniger als 30cm, vorzugsweise 20 cm oder 10 cm, vom elektrischen Bauteil angeordnet ist.

Mit der Kühleinrichtung können einzelne, mehrere, oder alle thermisch belasteten elektrischen Bauteile der Vorrichtung zur extrakorporalen Blutbehandlung gekühlt werden. Insbesondere können einzelne, mehrere, oder alle thermisch belasteten elektrischen Bauteile gekühlt werden, die eine Spezifikation aufweisen, dass sie nicht über 45°C oder 50°C erhitzt werden sollen oder die bei einer Heißdesinfektion oder Heißreinigung bei Betrieb der Vorrichtung ohne Kühlung einer Temperatur von mehr als 45°C oder 50°C oder 60°C ausgesetzt wären. In diesem Zusammenhang werden unter elektrischen Bauteilen sämtliche elektrische bzw. elektronische Bauelemente des Steuerungssystems verstanden. Unter dem Steuerungssystem wird die gesamte Elektronik der Vorrichtung zur extrakorporalen Blutbehandlung verstanden, d.h. sämtliche elektrische bzw. elektronische Bauelemente oder Bauteile, die auch einzelne Schaltungen oder Baugruppen bilden können. Die Kühleinrichtung kann auch mehrere Kühlkörper umfassen.

Für die Erfindung ist nicht wesentlich, wie der Kühlkörper ausgebildet ist, der mit dem mindestens einen Bauteil in einem thermischen Kontakt steht und mit der Flüssigkeit gekühlt wird. Entscheidend ist aber, dass der Kühlkörper mit der Flüssigkeit in einem thermischen Kontakt steht, so dass die Wärme abgeführt werden kann. Der Kühlkörper, der aus einem wärmeleitenden Material besteht, beispielsweise Aluminium, kann von der Flüssigkeit umspült und/oder von der Flüssigkeit durchspült werden. Beispielsweise können Kanäle in dem Kühlkörper ausgebildet sein. Die Flüssigkeit kann über einen oder mehrere Einlässe zuströmen und über einen oder mehrere Auslässe abströmen. Die Flüssigkeit kann dann verworfen werden. Die Flüssigkeit wird zu einem Ablauf geleitet, der ein zusätzlicher Ablauf oder der bei einer Dialysemaschine standardmäßig vorhandene Ablauf für das gebrauchte Dialysat sein kann.

Die Kühlung des mindestens einen Kühlkörpers mit der Flüssigkeit erfolgt während eines Betriebsmodus der Vorrichtung zur extrakorporalen Blutbehandlung, in dem die thermische Belastung am höchsten ist. Dieser Betriebsmodus ist die Heiß-Desinfektion des Hydrauliksystems, bei der auf eine vorgegebene Temperatur erwärmte Flüssigkeit durch mindestens einen Teil der Leitungen des Hydrauliksystems strömt. Die Erfindung macht in vorteilhafter Weise davon Gebrauch, dass bei der Dialysemaschine die Zuflussstrecke für die zentrale Flüssigkeitsversorgung mit einer Flüssigkeit, insbesondere Permeat, regelmäßig nicht von der Heiß-Desinfektions-Zirkulation erfasst wird. Folglich kann die Kühleinrichtung in die Zuflussstrecke oder in eine mit der Zuflussstrecke fluidisch verbundene Bypassleitung, die mit dem Abfluss verbunden sein kann, geschaltet werden. Die Kühleinrichtung kann daher von der Heiß-Desinfektion unabhängig betrieben werden. Durch eine solche Anordnung in der Zuflussstrecke kann zusätzlich erreicht werden, dass die Abwärme des elektrischen Bauteils die Kühlflüssigkeit erwärmt, die anschließend wenigstens teilweise oder vollständig in das Hydrauliksystem geleitet wird und dort nicht mehr so stark aufgeheizt werden muss, sei es für eine Behandlung sei es für eine Heißdesinfektion. So kann eine Energieeinsparung erzielbar sein.

Das Hydrauliksystem weist eine Flüssigkeits-Aufbereitungseinrichtung mit einem Einlass auf, wobei der Anschluss für den Zufluss einer Flüssigkeit über eine Zuflussleitung mit dem Einlass der Flüssigkeits-Aufbereitungseinrichtung verbunden ist. Diese Zuflussleitung bildet die Zuflussstrecke für die zentrale Flüssigkeitsversorgung. Die Flüssigkeit kann Dialysat sein, wobei die Flüssigkeits-Aufbereitungseinrichtung eine Dialysat-Aufbereitungseinrichtung ist.

In diesem Zusammenhang werden unter einer Flüssigkeits-Aufbereitungseinrichtung sämtliche Komponenten der Dialysemaschine verstanden, die der Aufbereitung einer Flüssigkeit, beispielsweise der Herstellung, Erwärmung oder Entgasung des Dialysats dienen. Die Flüssigkeits-Aufbereitungseinrichtung kann beispielsweise eine Eingangskammer zum Sammeln einer Flüssigkeit oder eine Entgasungskammer oder eine Heizvorrichtung etc. umfassen.

Wenn die Flüssigkeits-Aufbereitungseinrichtung eine Eingangskammer zum Sammeln einer Flüssigkeit aufweist, kann der Einlass der Flüssigkeits-Aufbereitungseinrichtung ein Einlass der Eingangskammer sein. Folglich umfasst die Zuflussstrecke nur den Bereich stromauf der Eingangskammer, so dass bei einer Unterbrechung oder Regulierung der Zufuhr von Flüssigkeit in die Eingangskammer unabhängig von der Zufuhr von Flüssigkeit zu der Kühleinrichtung eine Heiß-Desinfektion des Hydrauliksystems stattfinden kann. Bei einer besonders bevorzugten Ausführungsform sind in der Zuflussleitung Mittel zur Unterbrechung oder Regulierung der Flüssigkeitsströmung vorgesehen.

Unterschiedliche Ausführungsformen weisen unterschiedliche Konfigurationen für den Einbau der Kühleinrichtung in das bestehende Flüssigkeitssystem der Dialysemaschine vor.

Bei einer ersten Ausführungsform weist die Zuflussleitung einen ersten Abschnitt auf, der den Anschluss für den Zufluss einer Flüssigkeit mit dem Einlass der Kühleinrichtung verbindet und einen zweiten Abschnitt auf, der den Auslass der Kühleinrichtung mit dem Einlass der Flüssigkeits-Aufbereitungseinrichtung verbindet. Die Kühleinrichtung ist somit in die Zuflussleitung geschaltet. Während der Heiß-Desinfektion kann die Zufuhr von Flüssigkeit in das Hydrauliksystem unterbrochen werden, so dass die Flüssigkeit nur zu der Kühleinrichtung strömt. Der Auslass der Kühleinrichtung kann über eine BypassLeitung mit einem Ablauf in Fluidverbindung stehen, der ein zusätzlicher Ablauf oder ein bereits vorhandener Ablauf der Dialysemaschine sein kann. Zur Unterbrechung oder Regulierung der Flüssigkeitsströmung in der Bypass-Leitung sind Mittel vorgesehen, die Ventile oder Drosseln sein können. Bei den Mitteln zur Unterbrechung kann es sich auch um Pumpen oder um Kombinationen verschiedenen der genannten Mittel handeln.

Eine besonders bevorzugte Ausführungsform sieht vor, dass die Kühleinrichtung einen Temperatursensor zum Messen der Temperatur eines elektrischen Bauteils aufweist, das mit dem Kühlkörper in thermischen Kontakt steht, und eine Steuereinheit aufweist, die die Mittel zur Unterbrechung oder Regulierung der Flüssigkeitsströmung in der BypassLeitung derart ansteuert, dass die Temperatur unterhalb eines vorgegebenen Grenzwertes liegt. Die Steuereinheit der Kühleinrichtung kann auch Bestandteil der zentralen Steuerund Recheneinheit der Vorrichtung zur extrakorporalen Blutbehandlung sein.

Bei einer weiteren Ausführungsform verbindet ein erster Abschnitt einer zusätzlichen Kühlflüssigkeitsleitung den Anschluss für den Zulauf von Flüssigkeit oder die Zuflussleitung mit dem Einlass der Kühleinrichtung, wobei der Auslass der Kühleinrichtung über einen zweiten Abschnitt der Kühlflüssigkeitsleitung mit einem Ablauf in Fluidverbindung steht, der wieder ein zusätzlicher Ablauf oder ein bereits vorhandener Ablauf der Dialysemaschine sein kann. In der Kühlflüssigkeitsleitung sind vorzugsweise Mittel zur Unterbrechung oder Regulierung der Flüssigkeitsströmung vorgesehen. Die Kühlflüssigkeitsleitung stellt somit wieder eine Bypass-Leitung dar. Auch bei dieser Ausführungsform kann die Regulierung der Flüssigkeitsströmung in Abhängigkeit von der Temperatur des mindestens einen Bauteils erfolgen.

Bei einer weiteren alternativen Ausführungsform zweigt von der Zuflussleitung ein erster Abschnitt einer Spülflüssigkeitsleitung ab und führt zu dem Einlass der Kühleinrichtung, wobei der Auslass der Kühleinrichtung über einen zweiten Abschnitt der Spülflüssigkeitsleitung mit einem Ablauf in Fluidverbindung steht. Eine derartige Spülflüssigkeitsleitung kann bei einer Dialysemaschine zum Spülen und/oder Desinfizieren der Zuflussstrecke mit einer Flüssigkeit bereits vorhanden sein. Diese Leitung wird dann sowohl zum Spülen und/oder Desinfizieren der Zuflussstrecke als auch für den Zufluss der Flüssigkeit zu der Kühleinrichtung genutzt. Die Spülflüssigkeitsleitung ist somit wieder eine Bypass-Leitung. Auch hier kann wieder eine Temperaturregelung erfolgen.

Anstelle einer Temperaturregelung der Absperrorgane oder Ventile in den Leitungen können die Absperrorgane oder Ventile auch zeitgesteuert werden. Beispielsweise können die Absperrorgane oder Ventile für vorgegebene Zeitintervalle von der Steuereinheit ganz oder teilweise geöffnet bzw. geschlossen werden.

Im Folgenden wird die Erfindung unter Bezugnahme auf die Zeichnungen im Einzelnen beschrieben.

Es zeigen:
- Fig. 1: ein erstes Ausführungsbeispiel der erfindungsgemäßen Blutbehandlungsvorrichtung mit einer Kühleinrichtung in stark vereinfachter schematischer Darstellung,
- Fig. 2: ein zweites Ausführungsbeispiel der erfindungsgemäßen Blutbehandlungsvorrichtung,
- Fig. 3: ein drittes Ausführungsbeispiel der erfindungsgemäßen Blutbehandlungsvorrichtung und
- Fig. 4: ein viertes Ausführungsbeispiel der erfindungsgemäßen Blutbehandlungsvorrichtung.

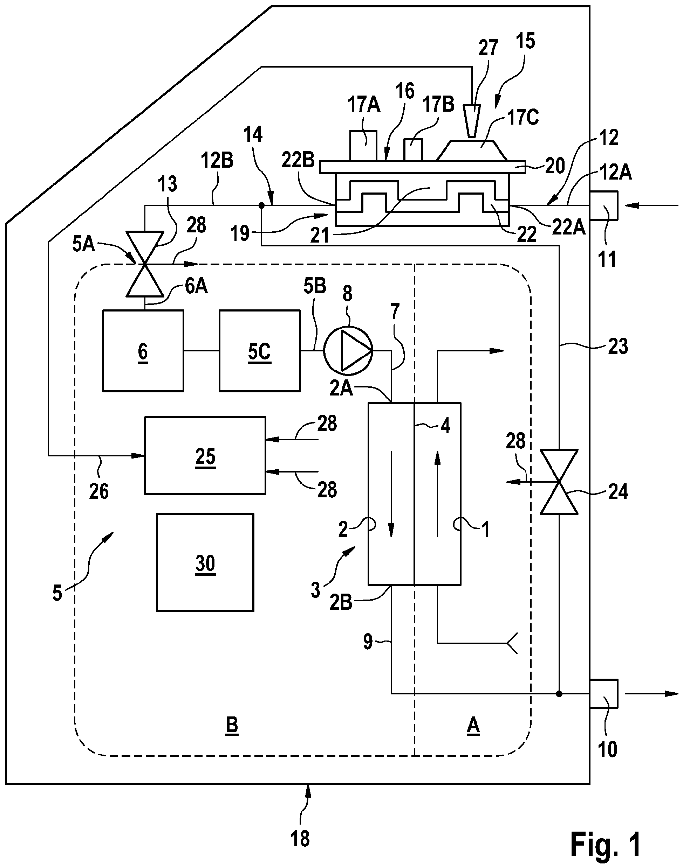

Fig. 1 zeigt ein Ausführungsbeispiel der erfindungsgemäßen Blutbehandlungsvorrichtung in stark vereinfachter schematischer Darstellung. Die Blutbehandlungsvorrichtung umfasst einen nur andeutungsweise dargestellten extrakorporalen Blutkreislauf A und ein Hydrauliksystem B. Der extrakorporale Blutkreislauf A schließt die Blutkammer 1 und das Hydrauliksystem B die Dialysatkammer 2 eines Dialysators 3 ein. Blutkammer 1 und Dialysatkammer 2 des Dialysators 3 sind durch eine semipermeable Membran 4 getrennt. Zur Bereitstellung des Dialysats weist das Hydrauliksystem B eine Flüssigkeits-Aufbereitungseinrichtung 5 auf, insbesondere Dialysat-Aufbereitungseinrichtung, die einen Einlass 5A für eine Flüssigkeit zur Herstellung des Dialysats, insbesondere Permeat (Reinwasser), und einen Auslass 5B aufweist. Das Permeat kann in einer Eingangskammer 6 gesammelt werden. Zur Herstellung des Dialysats wird das Permeat mit einem oder mehreren Konzentraten gemischt. Die übrigen Komponenten 5C der Dialysat-Aufbereitungseinrichtung 5 sind in Fig. 1 nur schematisch dargestellt. Von dem Auslass 5B der Dialysat-Aufbereitungseinrichtung 5 führt eine Dialysatzuführleitung 7 zu dem Einlass 2A der Dialysatkammer 2 des Dialysators 3. Das Dialysat wird mit einer Dialysatpumpe 8 gefördert. Der Auslass 2B der Dialysatkammer 2 ist über eine Dialysatabführleitung 9 mit einem Ablauf 10 für gebrauchtes Dialysat verbunden.

Diese Beschreibung betrifft eine Phase, bei der die Behandlung eines Patienten erfolgt. Während der Vorbereitung oder Nachbereitung der Behandlung braucht die Vorrichtung die Komponenten des extrakorporalen Blutkreislaufs A und den Dialysator 3 nicht aufzuweisen bzw. die Dialysatzuführleitung 7 kann mit der Dialysatabführleitung 9 unter Umgehung des Dialysators 3 kurgeschlossen sein. Zudem kann die Vorrichtung über Desinfektionsmittelkonzentratanschlüsse verfügen. Über die Desinfektionsmittelkonzentratanschlüsse kann konzentriertes Desinfektionsmittel dem Hydrauliksystem B zugeführt werden und durch Vermischen mit Wasser, das über eine Zuflussstrecke 14 dem Hydrauliksystem B zugeführt werden kann, die gewünschte Desinfektionsmittelkonzentration im Hydrauliksystem B zur Verfügung gestellt werden.

Das Permeat wird der Blutbehandlungsvorrichtung an einem zentralen Anschluss 11 zugeführt, der über eine Zuflussleitung 12 mit dem Einlass 6A der Eingangskammer 6 der Dialysat-Aufbereitungseinrichtung 5 verbunden ist. An dem Einlass 6A der Eingangskammer 6 ist ein Einlassventil 13 vorgesehen, so dass das Hydrauliksystem B von der Zuflussstrecke 14 abgetrennt werden kann.

In Fig. 1 sind nur die für die Erfindung wesentlichen Komponenten der Hydrauliksystems B dargestellt. Das Hydrauliksystem B kann noch weitere Komponenten 30, beispielsweise eine Bilanziereinheit, umfassen.

Darüber hinaus weist die Blutbehandlungsvorrichtung ein Steuerungssystem 15 auf, das mehrere Schaltungen mit elektrischen Bauteilen umfassen kann. In Fig. 1 ist nur eine Schaltung 16 mit mehreren Bauteile 17A, 17B, 17C dargestellt.

Die Blutbehandlungsvorrichtung sieht einen Betriebsmodus für eine Heiß-Desinfektion des Hydrauliksystems B vor, in dem das Hydrauliksystem mit der Flüssigkeit, die der Blutbehandlungsvorrichtung an dem zentralen Anschluss 11 zugeführt wird, gespült wird.

Hierzu wird die Flüssigkeit, insbesondere Permeat, auf eine Temperatur von größer als 80°C erwärmt. Während der Heiß-Desinfektion steigt die Lufttemperatur in dem Inneren Gehäuses 18 der Blutbehandlungsvorrichtung an, was zu einer thermischen Belastung der elektrischen Bauteile 17A, 17B, 17C führt.

Für die Kühlung der elektrischen Bauteile 17A, 17B, 17C ist eine Kühleinrichtung 19 vorgesehen, die für eine Ausführungsform nachfolgend beschrieben wird. Die elektrischen Bauteile17A, 17B, 17C befinden sich auf einer Platine 20, die auf einem Kühlkörper 21 montiert ist, so dass die Bauelemente mit dem Kühlkörper in einem thermischen Kontakt stehen. Der Kühlkörper 21 weist einen oder mehrere Kanäle 22 auf, die einen Einlass 22A und eine Auslass 22B haben, so dass der Kühlkörper von einer Flüssigkeit durchströmt werden kann, um von dem Kühlkörper die Wärme abzuführen. Als Kühlflüssigkeit für den Kühlkörper wird das zentral der Maschine zugeführte Permeat verwendet.

Bei dem vorliegenden Ausführungsbeispiel ist der Kühlkörper 21 in die Zuflussleitung 12 geschaltet. Die Zuflussleitung 12 umfasst einen ersten Abschnitt12A, der den Anschluss 11 mit dem Einlass 22A des Kühlkörpers 21 verbindet und einen zweiten Abschnitt 22B, der den Auslass 22B des Kühlkörpers 21 mit dem Einlass 6A der Eingangskammer 6 verbindet. Von dem zweiten Abschnitt 12B der Zuflussleitung 12 zweigt eine BypassLeitung 23 ab, die zu dem Ablauf 10 führt. In der Bypass-Leitung 23 ist ein Bypass-Ventil 24 vorgesehen.

Während der Heiß-Desinfektion kann das Einlass-Ventil 13 an der Eingangskammer 6 zumindest zeitweise geschlossen bleiben, so dass das Hydrauliksystem B von der Zuflusstrecke 14 abgetrennt ist. Das Bypass-Ventil 24 in der Bypass-Leitung 23 wird geöffnet, so dass kaltes Permeat zur Wärmeabfuhr in den Kühlkörper 21 strömt. Das Permeat fließt dann in den Ablauf 10 ab.

In einer Ausführungsform kann zur Steuerung der Flüssigkeitsströmung die Kühleinrichtung 19 eine Steuereinheit 25 aufweisen, die Bestandteil der Steuer- und Recheneinheit der Blutbehandlungsvorrichtung sein kann, d.h. Teil des Steuerungssystems ist. Die Steuereinheit 25 ist über eine Messleitung 26 mit einem Temperatursensor 27 verbunden, der die Temperatur eines elektrischen Bauteils17C der elektrischen Schaltung 16 misst, und ist über Steuerleitungen 28 mit dem Einlass-Ventil 13 und dem Bypass-Ventil 24 verbunden. Die Steuereinheit 25 vergleicht die mit dem Temperatursensor 27 gemessene Temperatur mit einem vorgegebenen Grenzwert, der unterhalb der zulässigen Betriebstemperatur des Bauteils liegt. Wenn die Temperatur oberhalb des Grenzwertes liegt, öffnet die Steuereinheit 25 das Bypass-Ventil 24, so dass zur Wärmeabfuhr Permeat durch den Kühlkörper 21 strömt. Wenn die Temperatur unterhalb des Grenzwertes liegt, schließt die Steuereinheit 25 das Bypass-Ventil 24, so dass die Flüssigkeitsströmung unterbrochen ist. Anstelle eines Ventils kann auch eine Drossel in der Bypass-Leitung 23 vorgesehen sein, die einen bestimmen Durchfluss vorgibt. Die Steuereinheit 25 kann die Flüssigkeitsströmung in das Hydrauliksystem B auch durch nur teilweises Öffnen und Schließen des Einlass-Ventils 13 regulieren. Die Steuereinheit 25 kann die Flüssigkeitsströmung in der Zuflussleitung 12 auch derart steuern, dass Flüssigkeit primär in das Hydrauliksystem B strömt, wenn ein solcher Fluss auf Grund einer Anforderung durch eine Hydrauliksteuerung angefordert ist. Wenn dieser Fluss für die Kühlung nicht ausreichen sollte, kann die Steuereinheit 25 als sekundäre Maßnahme den Fluss über die Bypassleitung 23 veranlassen, beispielsweise durch Öffnen des Ventils 24.

In allen hier beschriebenen Ausführungsformen kann in der Vorrichtung eine in den Figuren nicht gezeigte Pumpe vorgesehen sein, mit der die Flüssigkeit durch die BypassLeitung gepumpt werden kann. Bei dieser Pumpe kann es sich um eine peristaltische Pumpe oder eine Membranpumpe handeln. In diesem Fall kann das Ventil 24 die peristaltische Pumpe oder die Membranpumpe sein.

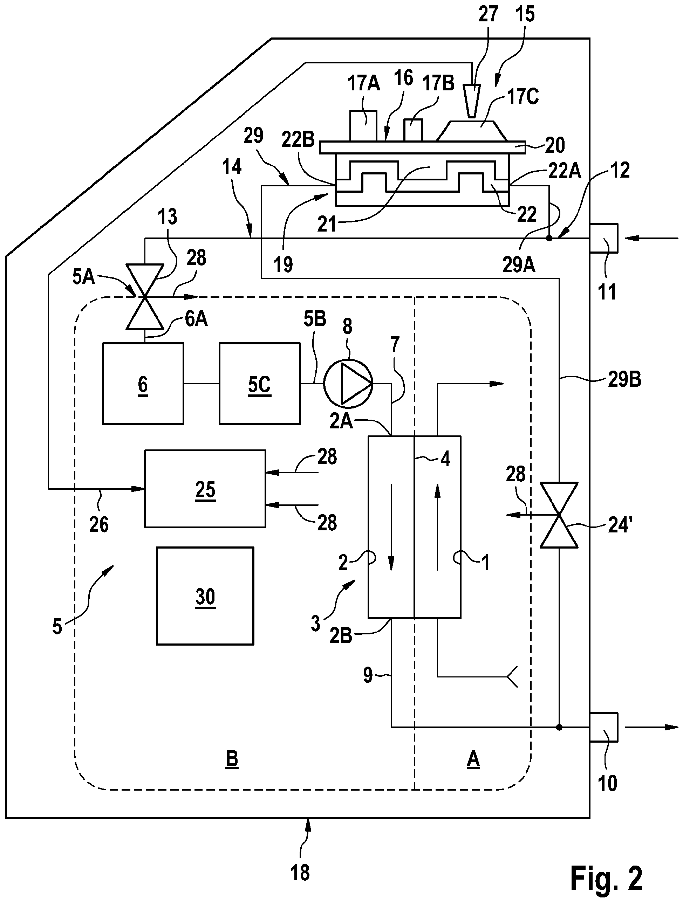

Fig. 2 zeigt ein zweites Ausführungsbeispiel der Blutbehandlungsvorrichtung, das sich von der ersten Ausführungsform durch den Einbau der Kühleinrichtung 19 unterscheidet. Die einander entsprechenden Teile sind mit denselben Bezugszeichen versehen. Bei dem zweiten Ausführungsbeispiel ist der Kühlkörper 21 nicht in die Zuflussleitung 12 geschaltet. Von der Zuflussleitung 12 zweigt ein erster Abschnitt 29A einer zusätzlichen Kühlflüssigkeitsleitung 29 ab, die zu dem Einlass des Kühlkörpers 21 führt. Ein zweiter Abschnitt 29B der Kühlflüssigkeitsleitung 29 verbindet den Auslass 22A des Kühlkörpers 21 mit dem Ablauf 10. Zur Regulierung des Kühlmittelflusses ist in dem zweiten Abschnitt 29B der Kühlflüssigkeitsleitung 29 ein Ventil 24` vorgesehen. Das Ventil 24` in der Kühlflüssigkeitsleitung 29 kann von Steuereinheit 25 wie das Bypass-Ventil 24 in der Bypass-Leitung 23 des ersten Ausführungsbeispiels in Abhängigkeit von der mit dem Temperatursensor 27 gemessenen Temperatur geregelt werden.

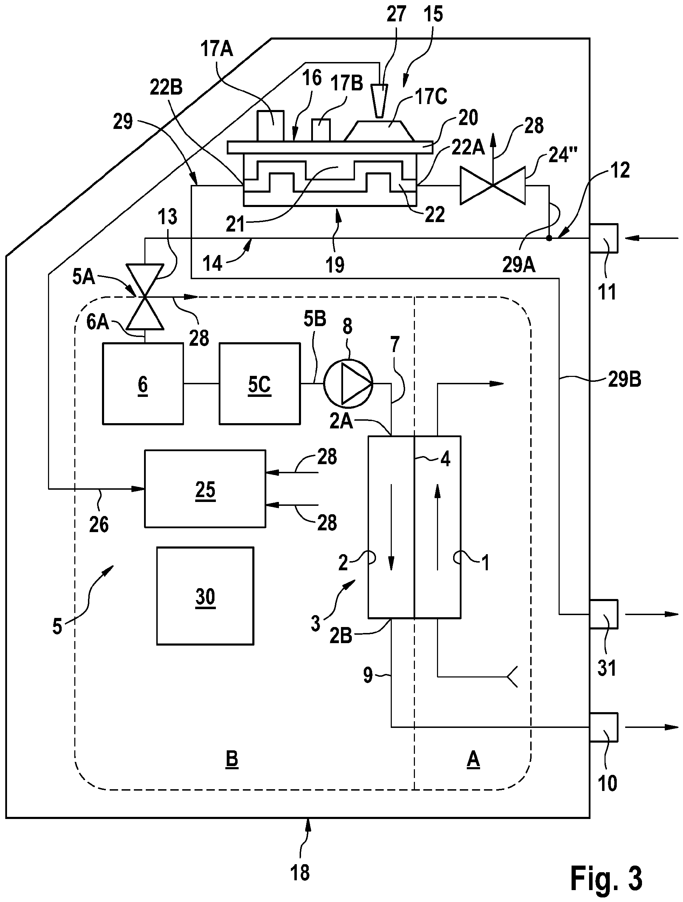

Die Fig. 3 zeigt ein drittes Ausführungsbeispiel der Blutbehandlungsvorrichtung, das sich von der zweiten Ausführungsform dadurch unterscheidet, dass das Ventil 24" zur Steuerung der Flüssigkeitsströmung nicht in dem zweiten Abschnitt 29B, sondern dem ersten Abschnitt 29A der Kühlflüssigkeits-Leitung angeordnet ist. Die Regelung der Flüssigkeitsströmung kann wie bei dem zweiten Ausführungsbeispiel erfolgen. Darüber hinaus unterscheidet sich das dritte Ausführungsbeispiel von der zweiten Ausführungsform dadurch, dass der zweite Abschnitt 29B der Kühlflüssigkeits-Leitung 29 nicht zu dem Ablauf für gebrauchtes Dialysat, sondern zu einem zusätzlichen Ablauf 31 für das Kühlmittel führt. Der zweite Abschnitt 29B der Kühlflüssigkeits-Leitung 29 kann aber auch bei dem dritten Ausführungsbeispiel an dem Ablauf 10 für gebrauchtes Dialysat angeschlossen sein (Figuren 1 und 2). Das dritte Ausführungsbeispiel mit dem separaten Abfluss 31 für das Kühlmittel hat den Vorteil, dass eine Kontamination der Zuflussstrecke 14 durch gebrauchtes Dialysat aus dem Ablauf 10 ausgeschlossen ist.

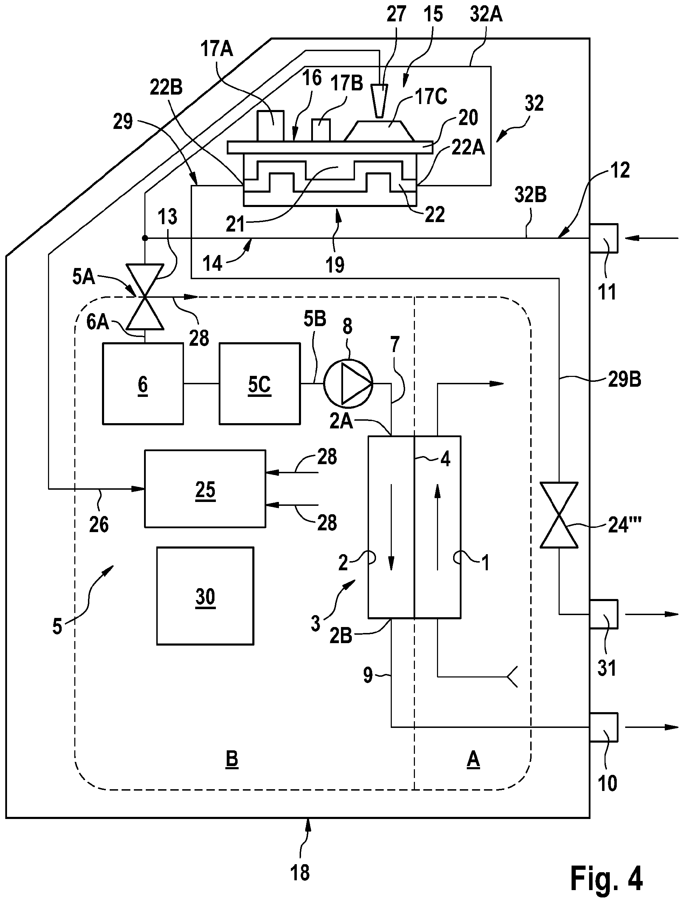

Fig. 4 zeigt eine weitere alternative Ausführungsform einer Blutbehandlungsvorrichtung, die eine Spülung bzw. Desinfektion der Zuflussstrecke 14 vorsieht. Für die Spülung bzw. Desinfektion ist bei einer derartigen Blutbehandlungsvorrichtung eine Spülflüssigkeitsleitung 32 vorgesehen, die von der Zuflussleitung 12 stromauf des Einlasses 5A der Dialysat-Aufbereitungseinrichtung 5, insbesondere am Ende der Zuflussstrecke 14, beispielsweise stromauf des Einlass-Ventils 13 und vorzugsweise nahe dem Einlass-Ventil 13 von der Zuflussleitung 12 abzweigt und zu einem separaten Ablauf 31 führt. Der Kühlkörper 21 ist bei der alternativen Ausführungsform in die Spülflüssigkeitsleitung 32 geschaltet. Die Spülflüssigkeitsleitung 32 umfasst einen ersten Abschnitt 32A, der von der Zuflussleitung 12 abzweigt und zu dem Einlass 22A des Kühlkörpers 21 führt und einen zweiten Abschnitt 32B, der den Auslass 22B des Kühlkörpers 21 mit dem separaten Ablauf 31 verbindet. Das Ventil 24‴ zur Regulierung des Kühlmittelflusses kann in dem zweiten Abschnitt 32B der Spülflüssigkeitsleitung 32 vorgesehen sein. Bei diesem Ausführungsbeispiel wird also für die Zuführung des Kühlmittels von einer bereits vorhandenen Leitung Gebrauch gemacht, die ansonsten für die Zuführung einer Spülflüssigkeit verwendet wird. Die Steuerung der Ventile 13 und 24‴ kann wie bei den obigen Ausführungsformen erfolgen.

## Patentansprüche

1. Vorrichtung zur extrakorporalen Blutbehandlung mit einem mehrere Leitungen (7, 9) umfassenden Hydrauliksystem (B) zur Bereitstellung von Dialysat für eine Dialysebehandlung und einem mindestens ein elektrisches Bauteil (17A, 17B, 17C) umfassenden Steuerungssystem (15), wobei das Hydrauliksystem (B) mindestens einen Anschluss (11) für den Zufluss einer Flüssigkeit in die Vorrichtung zur extrakorporalen Blutbehandlung aufweist, wobei
die Vorrichtung zur extrakorporalen Blutbehandlung eine Kühleinrichtung (19) zum Kühlen des elektrischen Bauteils oder mindestens eines der elektrischen Bauteile (17A, 17B, 17C) des Steuerungssystems (15) aufweist, welche mindestens einen mit einer Flüssigkeit kühlbaren Kühlkörper (21) aufweist, der mit mindestens einem elektrischen Bauteil (17A, 17B, 17C) in einem thermischen Kontakt steht, wobei die Kühleinrichtung (19) mindestens einen Einlass (22A) aufweist, der mit dem Anschluss (11) für den Zufluss einer Flüssigkeit in Fluidverbindung steht, und mindestens einen Auslass (22B) aufweist, der mit einem Ablauf (10, 31) in Fluidverbindung steht,
**dadurch gekennzeichnet, dass**
das Hydrauliksystem (B) eine Flüssigkeits-Aufbereitungseinrichtung (5) mit einem Einlass (5A) aufweist, wobei der Anschluss (11) für den Zufluss einer Flüssigkeit über eine Zuflussleitung (12) mit dem Einlass (5A) der Flüssigkeits-Aufbereitungseinrichtung (5) verbunden ist, und wobei von der Zuflussleitung (12) eine Bypassleitung (23, 29, 32) abgeht, die in Fluidverbindung mit dem Ablauf (10, 31) steht.

2. Vorrichtung zur extrakorporalen Blutbehandlung nach Anspruch 1, **dadurch gekennzeichnet, dass** die Flüssigkeits-Aufbereitungseinrichtung (5) eine Eingangskammer (6) zum Sammeln einer Flüssigkeit aufweist, wobei der Einlass (5A) der Flüssigkeits-Aufbereitungseinrichtung (5) ein Einlass (6A) der Eingangskammer (6) ist.

3. Vorrichtung zur extrakorporalen Blutbehandlung nach Anspruch 1 oder 2, **dadurch gekennzeichnet, dass** Mittel (13) zur Unterbrechung oder Regulierung der Flüssigkeitsströmung in der Zuflussleitung (12) stromab der Abzweigung der Bypassleitung (23, 29, 32) von der Zuflussleitung (12) vorgesehen sind.

4. Vorrichtung zur extrakorporalen Blutbehandlung nach Anspruch 1 bis 3, **dadurch gekennzeichnet, dass** die Zuflussleitung (12) einen ersten Abschnitt (12A) aufweist, der den Anschluss (11) für den Zufluss einer Flüssigkeit mit dem Einlass (22A) der Kühleinrichtung (19) verbindet und einen zweiten Abschnitt (12B) aufweist, der den Auslass (22B) der Kühleinrichtung (19) mit dem Einlass (5A) der Flüssigkeits-Aufbereitungseinrichtung (5) verbindet.

5. Vorrichtung zur extrakorporalen Blutbehandlung nach Anspruch 1 oder 2, **dadurch gekennzeichnet, dass** ein erster Abschnitt (29A) der Bypassleitung (29) den Anschluss (11) für den Zulauf von Flüssigkeit oder die Zuflussleitung (12) mit dem Einlass (22A) der Kühleinrichtung (19) verbindet, wobei der Auslass (22B) der Kühleinrichtung (19) über einen zweiten Abschnitt (29B) der Bypassleitung (29) mit einem Ablauf (10, 31) in Fluidverbindung steht.

6. Vorrichtung zur extrakorporalen Blutbehandlung nach einem der Ansprüche 1 bis 5, **dadurch gekennzeichnet, dass** Mittel (24, 24', 24") zur Unterbrechung oder Regulierung der Flüssigkeitsströmung in der Bypass-Leitung (23, 29, 32) vorgesehen sind.

7. Vorrichtung zur extrakorporalen Blutbehandlung nach Anspruch 6, **dadurch gekennzeichnet, dass** die Kühleinrichtung (19) einen Temperatursensor (27) zum Messen der Temperatur eines elektrischen Bauteils (17A, 17B, 17C) aufweist, das mit dem Kühlkörper (21) in thermischen Kontakt steht, und eine Steuereinheit (25) aufweist, die die Mittel (24, 24', 24") zur Unterbrechung oder Regulierung der Flüssigkeitsströmung in der Bypass-Leitung (23, 29, 32) derart ansteuert, dass die Temperatur unterhalb eines vorgegebenen Grenzwertes liegt.

8. Vorrichtung zur extrakorporalen Blutbehandlung nach einem der Ansprüche 1 bis 7, **dadurch gekennzeichnet, dass** der Ablauf (31) von einem weiteren Ablauf (10) für das Hydrauliksystem (B), insbesondere von einem Ablauf für Dialysat während der Behandlung, unabhängig ist.

9. Verfahren zum Betreiben einer Vorrichtung zur extrakorporalen Blutbehandlung in einem Betriebsmodus zur Durchführung einer Heiß-Desinfektion, wobei die Vorrichtung zur extrakorporalen Blutbehandlung ein mehrere Leitungen umfassendes Hydrauliksystem zur Bereitstellung von Dialysat für einen Dialysator und ein mindestens ein elektrisches Bauteil umfassendes Steuerungssystem aufweist, wobei die Vorrichtung zur extrakorporalen Blutbehandlung im Betriebsmodus zur Durchführung der Heiß-Desinfektion die Durchführung der Heiß-Desinfektion des Hydrauliksystems vorsieht, bei der eine auf eine vorgegebene Temperatur erwärmte Flüssigkeit durch mindestens einen Teil der Leitungen des Hydrauliksystem strömt, **dadurch gekennzeichnet, dass** während des Betriebsmodus der Heiß-Desinfektion mindestens ein Kühlkörper der mit mindestens einem elektrischen Bauteil des Steuerungssystems in einem thermischen Kontakt steht, mit einer Flüssigkeit gekühlt wird, die der Vorrichtung zur extrakorporalen Blutbehandlung zugeführt wird, während die auf eine vorgegebene Temperatur erwärmte Flüssigkeit durch mindestens einen Teil der Leitungen des Hydrauliksystem strömt.

10. Verfahren nach Anspruch 9, **dadurch gekennzeichnet, dass** die für die HeißDesinfektion verwendete Flüssigkeit Permeat ist.

11. Verfahren nach Anspruch 9 oder 10, **dadurch gekennzeichnet, dass** die Temperatur des mindestens einen Bauteils gemessen wird, und der Zufluss der Flüssigkeit, die der Vorrichtung zur extrakorporalen Blutbehandlung zugeführt wird, derart geregelt wird, dass die Temperatur des mindestens einen Bauteils unterhalb eines vorgegebenen Grenzwertes liegt.

12. Verfahren nach einem der Ansprüche 9 bis 11, **dadurch gekennzeichnet, dass** zur Durchführung des Verfahrens eine Vorrichtung nach einem der Ansprüche 1 bis 8 verwendet wird.

## Claims

1. Device for extracorporeal blood treatment, comprising a hydraulic system (B) that has a plurality of lines (7, 9) and is intended for providing dialysate for a dialysis treatment, and a control system (15) comprising at least one electrical component (17A, 17B, 17C), the hydraulic system (B) having at least one port (11) for supplying a fluid to the device for extracorporeal blood treatment,
the device for extracorporeal blood treatment having a cooling apparatus (19) for cooling the electrical component or at least one of the electrical components (17A, 17B, 17C) of the control system (15), which cooling apparatus has at least one heat sink (21) that can be cooled by a fluid and is in thermal contact with at least one electrical component (17A, 17B, 17C), the cooling apparatus (19) having at least one inlet (22A) that is in fluid connection with the port (11) for supplying a fluid, and having at least one outlet (22B) that is in fluid connection with a drain (10, 31),
**characterized in that**,
the hydraulic system (B) has a fluid-preparation apparatus (5) having an inlet (5A), the port (11) for supplying a fluid via a fluid line (12) being connected to the inlet (5A) of the fluid-preparation apparatus (5), and a bypass line (23, 29, 32) coming off the supply line (12), which bypass line is in fluid connection with the drain (10, 31).

2. Device for extracorporeal blood treatment according to claim 1, **characterised in that** the fluid-preparation apparatus (5) has an input chamber (6) for collecting a fluid, the inlet (5A) of the fluid-preparation apparatus (5) being an inlet (6A) of the input chamber (6).

3. Device for extracorporeal blood treatment according to either claim 1 or claim 2, **characterised in that** means (13) for interrupting or adjusting the fluid flow in the supply line (12) are provided downstream of the branch-off of the bypass line (23, 29, 32) from the supply line (12).

4. Device for extracorporeal blood treatment according to claims 1 to 3, **characterised in that** the supply line (12) comprises a first portion (12A) that connects the port (11) for supplying a fluid to the inlet (22A) of the cooling apparatus (19), and a second portion (12B) that connects the outlet (22B) of the cooling apparatus (19) to the inlet (5A) of the fluid-preparation apparatus (5).

5. Device for extracorporeal blood treatment according to either claim 1 or claim 2, **characterised in that** a first portion (29A) of the bypass line (29) connects the port (11) for supplying fluid or the supply line (12) to the inlet (22A) of the cooling apparatus (19), the outlet (22B) of the cooling apparatus (19) being in fluid connection with a drain (10, 31) via a second portion (29B) of the bypass line (29).

6. Device for extracorporeal blood treatment according to any of claims 1 to 5, **characterised in that** means (24, 24', 24") for interrupting or adjusting the fluid flow are provided in the bypass line (23, 29, 32).

7. Device for extracorporeal blood treatment according to claim 6, **characterised in that** the cooling apparatus (19) has a temperature sensor (27) for measuring the temperature of an electrical component (17A, 17B, 17C) that is in thermal contact with the heat sink (21), and a control unit (25) that actuates the means (24, 24', 24") for interrupting or adjusting the fluid flow in the bypass line (23, 29, 32) such that the temperature is below a specified threshold value.

8. Device for extracorporeal blood treatment according to any of claims 1 to 7, **characterised in that** the drain (31) is independent of an additional drain (10) for the hydraulic system (B), in particular of a drain for dialysate during treatment.

9. Method for operating a device for extracorporeal blood treatment during an operating mode for carrying out a hot-water disinfection, wherein the device for extracorporeal blood treatment comprises a hydraulic system that has a plurality of lines and is intended for providing dialysate for a dialyser, and a control system having at least one electrical component, the device for extracorporeal blood treatment carrying out the hot-water disinfection of the hydraulic system in the operating mode for carrying out the hot-water disinfection, in which a fluid heated to a specified temperature flows through at least some of the lines of the hydraulic system, **characterised in that**, during the hot-water disinfection operating mode, at least one heat sink which is in thermal contact with at least one electrical component of the control system is cooled using a fluid which is supplied to the device for extracorporeal blood treatment, while the fluid heated to a specified temperature flows through at least some of the lines of the hydraulic system.

10. Method according to claim 9, **characterised in that** the fluid used for hot-water disinfection is permeate.

11. Method according to either claim 9 or 10, **characterised in that** the temperature of the at least one component is measured, and the supply of the fluid which is supplied to the device for extracorporeal blood treatment is adjusted such that the temperature of the at least one component is below a specified threshold value.

12. Method according to any of claims 9 to 11, **characterised in that** a device according to any of claims 1 to 8 is used to carry out the method.

## Revendications

1. Dispositif de traitement extracorporel du sang avec un système hydraulique (B) comprenant plusieurs conduites (7, 9) pour la fourniture de dialysat pour un traitement par dialyse et un système de commande (15) comprenant au moins un composant électrique (17A, 17B, 17C), dans lequel le système hydraulique (B) présente au moins un raccord (11) pour l'admission d'un liquide dans le dispositif de traitement extracorporel du sang, dans lequel
le dispositif de traitement extracorporel du sang présente un dispositif de refroidissement (19), servant pour le refroidissement du composant électrique ou au moins un des composants électriques (17A, 17B, 17C) du système de commande (15), et qui présente au moins un corps de refroidissement (21) pouvant être refroidi avec un liquide, ce corps étant dans un contact thermique avec au moins un composant électrique (17A, 17B, 17C),
dans lequel le dispositif de refroidissement (19) présente au moins une entrée (22A) qui est en communication fluidique avec le raccord (11) pour l'admission d'un liquide, et au moins une sortie (22B) qui est en communication fluidique avec une sortie (10, 31),
**caractérisé en ce que**
le système hydraulique (B) présente un dispositif de préparation de liquide (5) avec une entrée (5A), dans lequel le raccord (11) est relié pour l'admission d'un liquide par le biais d'une conduite d'admission (12) à l'entrée (5A) du dispositif de préparation de liquide (5), et dans lequel une conduite de dérivation (23, 29, 32) qui s'étend depuis la conduite d'admission (12) est en communication fluidique avec la sortie (10, 31).

2. Dispositif de traitement extracorporel du sang selon la revendication 1, **caractérisé en ce que** le dispositif de préparation de liquide (5) présente une chambre d'entrée (6) pour la collecte d'un liquide, dans lequel l'entrée (5A) du dispositif de préparation de liquide (5) est une entrée (6A) de la chambre d'entrée (6).

3. Dispositif de traitement extracorporel du sang selon la revendication 1 ou 2, **caractérisé en ce que** des moyens (13) d'interruption ou de régulation de l'écoulement liquide dans la conduite d'admission (12) sont prévus en aval de la bifurcation de la conduite de dérivation (23, 29, 32) de la conduite d'admission (12).

4. Dispositif de traitement extracorporel du sang selon une des revendications 1 à 3, **caractérisé en ce que** la conduite d'admission (12) présente une première section (12A) qui relie le raccord (11) pour l'admission d'un liquide à l'entrée (22A) du dispositif de refroidissement (19) et présente une seconde section (12B) qui relie la sortie (22B) du dispositif de refroidissement (19) à l'entrée (5A) du dispositif de préparation de liquide (5).

5. Dispositif de traitement extracorporel du sang selon la revendication 1 ou 2, **caractérisé en ce qu'**une première section (29A) de la conduite de dérivation (29) relie le raccord (11) pour l'admission de liquide ou la conduite d'admission (12) à l'entrée (22A) du dispositif de refroidissement (19), dans lequel la sortie (22B) du dispositif de refroidissement (19) est en liaison fluidique par le biais d'une seconde section (29B) de la conduite de dérivation (29) avec une sortie (10, 31).

6. Dispositif de traitement extracorporel du sang selon l'une quelconque des revendications 1 à 5, **caractérisé en ce que** des moyens (24, 24', 24") d'interruption ou de régulation de l'écoulement de liquide sont prévus dans la conduite de dérivation (23, 29, 32).

7. Dispositif de traitement extracorporel du sang selon la revendication 6, **caractérisé en ce que** le dispositif de refroidissement (19) présente un capteur de température (27) pour la mesure de la température d'un composant électrique (17A, 17B, 17C) qui est en contact thermique avec le corps de refroidissement (21), et une unité de commande (25) qui commande les moyens (24, 24', 24") d'interruption ou de régulation de l'écoulement de liquide dans la conduite de dérivation (23, 29, 32) de telle manière que la température se trouve en dessous d'une valeur limite prédéfinie.

8. Dispositif de traitement extracorporel du sang selon l'une quelconque des revendications 1 à 7, **caractérisé en ce que** la sortie (31) est indépendante d'une autre sortie (10) pour le système hydraulique (B), en particulier d'une sortie pour du dialysat pendant le traitement.

9. Procédé de fonctionnement d'un dispositif de traitement extracorporel du sang dans un mode de fonctionnement pour la réalisation d'une désinfection à chaud, le dispositif de traitement extracorporel du sang présentant un système hydraulique comprenant plusieurs conduites afin de fournir en dialysat un dialyseur, et un système de commande comprenant au moins un composant électrique, dans lequel le dispositif de traitement extracorporel du sang prévoit dans le mode de fonctionnement pour la réalisation de la désinfection à chaud la réalisation de la désinfection à chaud du système hydraulique, pour laquelle un liquide chauffé à une température prédéfinie s'écoule par au moins une partie des conduites du système hydraulique, **caractérisé en ce que** pendant le mode de fonctionnement de désinfection à chaud, au moins un corps de refroidissement, qui est dans un contact thermique avec au moins un composant électrique du système de commande, est refroidi avec un liquide qui est fourni au dispositif de traitement extracorporel du sang, alors que le liquide chauffé à une température prédéfinie s'écoule par au moins une partie des conduites du système hydraulique.

10. Procédé selon la revendication 9, **caractérisé en ce que** le liquide utilisé pour la désinfection à chaud est du perméat.

11. Procédé selon la revendication 9 ou 10, **caractérisé en ce que** la température d'au moins un composant est mesurée, et l'admission du liquide qui est amené au dispositif de traitement extracorporel du sang, est régulée de telle manière que la température d'au moins un composant se trouve en dessous d'une valeur limite prédéfinie.

12. Procédé selon l'une quelconque des revendications 9 à 11, **caractérisé en ce qu'**un dispositif selon l'une quelconque des revendications 1 à 8 est utilisé pour la réalisation du procédé.
